# EUROPÄISCHE PATENTANMELDUNG

(11) **EP 2 241 273 A1**
(43) Veröffentlichungstag der Anmeldung: **20.10.2010**
(21) Anmeldenummer: 09005363.8
(22) Anmeldetag: 15.04.2009
(51) Int. Cl.: A61B 17/29, A61B 17/00, A61B 10/02, A61B 10/06, A61B 19/00

(54) **Zerlegbares chirurgisches Instrument**

(71) Anmelder: EFS - Eberle-Feinwerktechnische Systeme GmbH & Co. KG, 75449 Wurmberg (DE)
(72) Erfinder: Schniebs, Udo, 73614 Schorndorf (DE)
(74) Vertreter: Schaeberle, Steffen

(57) **Zusammenfassung**

Die Erfindung betrifft ein Chirurgisches Instrument, umfassend ein Außenrohr (2), ein Innenrohr (3), welches beweglich im Außenrohr (2) angeordnet ist und vollständig vom Außenrohr (2) lösbar ist, und eine chirurgische Schneidvorrichtung (4) zum Abtrennen und/oder Greifen von Körpergewebe oder dergleichen, und eine lösbare Verzahnungsverbindung (8) mit einem Zahnradelement (9) und einer Zahnstange (10) wobei die Schneidvorrichtung (4) eine schwenkbare Schneidbacke (5) und eine stationäre Schneidbacke (15) umfasst, und die schwenkbare Schneidbacke (5) um eine Achse (7) schwenkbar am Außenrohr (2) angeordnet ist, wobei die schwenkbare Schneidbacke (5) mittels der Verzahnungsverbindung (8) mit dem Innenrohr (3) verbunden ist und durch eine axiale Bewegung des Innenrohrs (3) betätigbar ist, und wobei das abgetrennte Gewebe durch das Innenrohr (3) hindurch absaugbar ist.

## Beschreibung

Die vorliegende Erfindung betrifft ein zerlegbares chirurgisches Instrument, welches ein vereinfachtes Reinigen ermöglicht.

Chirurgische Instrumente sind aus dem Stand der Technik in unterschiedlichen Ausgestaltungen bekannt und werden insbesondere bei der endoskopischen Chirurgie verwendet. Derartige chirurgische Instrumente weisen üblicherweise ein langgestrecktes rohrförmiges Element auf, welches an einem distalen Ende eine Öffnung zur Aufnahme von Körpermaterial aufweist. Aus der EP 0 590 887 B1 ist ein derartiges chirurgisches Instrument bekannt, welches ein inneres rotierbares Schneidwerkzeug aufweist, das über einen in einem Handstück angeordneten Antrieb betätigbar ist. Ferner sind manuell betätigbare chirurgische Instrumente bekannt, welche an ihrem distalen Ende eine Schneidvorrichtung aufweisen, um Gewebe o.Ä. abzutrennen. Derartige chirurgische Instrumente werden auch als "Stanzinstrumente" oder "Punches" bezeichnet. Insbesondere bei den handbetätigten Stanzinstrumenten hat sich herausgestellt, dass eine Reinigung zwischen einem Außenrohr und einem Innenrohr aufgrund mangelnder Zerlegbarkeit des Instruments sehr aufwendig bzw. nicht immer vollständig möglich ist. Diese mangelhafte Hygiene kann bei nachfolgenden Eingriffen, bei denen das Instrument verwendet wird, zu Komplikationen führen.

Es ist daher Aufgabe der vorliegenden Erfindung, bei einfachem Aufbau und einfacher, kostengünstiger Herstellbarkeit ein zerlegbares chirurgisches Instrument zu schaffen, das eine einfache und komplette Reinigung ermöglicht.

Diese Aufgabe wird durch ein zerlegbares chirurgisches Instrument mit den Merkmalen des Anspruchs 1 gelöst. Die Unteransprüche zeigen bevorzugte Weiterbildungen der Erfindung.

Das erfindungsgemäße zerlegbare chirurgische Instrument mit den Merkmalen des Anspruchs 1 weist den Vorteil auf, dass es bei einfachem Aufbau eine sehr gute und einfache Reinigung ermöglicht. Dies wird erfindungsgemäß dadurch erreicht, dass ein hohles Innenrohr aus einem Außenrohr entnehmbar ist, wobei das Innenrohr mit einer am Außenrohr angeordneten Schneidvorrichtung über eine lösbare Verzahnungsverbindung verbunden ist. Die Verzahnungsverbindung umfasst ein Zahnradelement und eine Zahnstange, so dass eine lösbare Verbindung zwischen dem Innenrohr und der Schneidvorrichtung vorhanden ist sobald Zahnstange außer Eingriff von dem Zahnradelement kommt. Abgetrenntes Körpermaterial kann dabei durch das hohle Innenrohr abgesaugt werden. Nach einer Operation kann dann das Innenrohr aus dem Außenrohr entfernt werden und eine einfache und sichere Reinigung der Bauteile des chirurgischen Instruments sichergestellt werden.

Für einen besonders einfachen Aufbau ist vorzugsweise die Zahnstange im Innenrohr und das Zahnradelement der Verzahnungsverbindung an der Schneidvorrichtung angeordnet.

Besonders bevorzugt umfasst die Schneidvorrichtung eine Schneidbacke mit einem distalen und einem proximalen Bereich. Der distale Bereich weist mindestens eine Schneide auf und ist länger als der proximale Bereich ausgebildet.

Ferner weist das Innenrohr vorzugsweise einen Kontaktbereich auf, welcher mit dem proximalen Bereich der Schneidbacke in Kontakt bringbar ist, um die Verzahnungsverbindung außer Eingriff zu bringen. Um ein möglichst einfaches Außereingriffbringen zu ermöglichen, weist der Kontaktbereich am Innenrohr bevorzugt eine Auflaufschräge auf. Besonders bevorzugt ist die Auflaufschräge ballig mit einem Radius, um einen feinfühligen Kontakt zwischen der Auflaufschräge und der Schneidbacke zu ermöglichen. Der Radius der Auflaufschräge geht dabei besonders bevorzugt in einen linearen Bereich über, wobei der Radius vorzugsweise größer als ein Radius eines Außenumfangs des Innenrohrs ist. Bevorzugt liegt ein Verhältnis des Radius der Auflaufschräge zu einem Radius des Außendurchmessers des Innenrohrs im Bereich von 1,1 bis 1,6 und beträgt vorzugsweise 1,49.

Weiter bevorzugt ist zwischen der Zahnstange und dem Kontaktbereich eine Ausnehmung zur Aufnahme des proximalen Bereichs der Schneidbacke bei einem Schwenkvorgang vorgesehen. Gemäß einer weiteren bevorzugten Ausgestaltung der Erfindung ist ein Spülrohr vorgesehen, welches an der Außenseite des Außenrohrs, vorzugsweise am oberen Bereich des Außenrohrs, angeordnet ist.

Gemäß einer weiteren bevorzugten Ausgestaltung der Erfindung umfasst das chirurgische Instrument eine Kupplung, welche am Außenrohr angeordnet ist, um eine Verbindung mit einem insbesondere manuell betätigbaren Handstück herzustellen. Hierdurch kann das chirurgische Instrument vom Handstück getrennt werden und dann für die Reinigung auseinandergebaut werden. Besonders bevorzugt weist die Kupplung eine Vielzahl von Bohrungen entlang des Außenumfangs des Außenrohrs auf. Hierdurch kann eine Verbindung mit dem Handstück in verschiedenen Positionen erfolgen, wobei das chirurgische Instrument dadurch immer optimal an die jeweilige Situation angepasst werden kann.

Nachfolgend wird unter Bezugnahme auf die begleitende Zeichnung ein Ausführungsbeispiel der Erfindung im Detail beschrieben. In der Zeichnung ist:
- Fig. 1: eine schematische, perspektivische Ansicht eines chirurgischen Instruments gemäß einem Ausführungsbeispiel der Erfindung,
- Fig. 2: eine schematische, perspektivische Ansicht des in Fig. 1 gezeigten Instru- ments in einem auseinandergebauten Zustand,
- Fig. 3 bis 6: schematische Schnittansichten des chirurgischen Instruments in verschie- denen Stellungen und
- Fig. 7 bis 10: schematische, perspektivische Ansichten des chirurgischen Instruments in verschiedenen Stellungen.

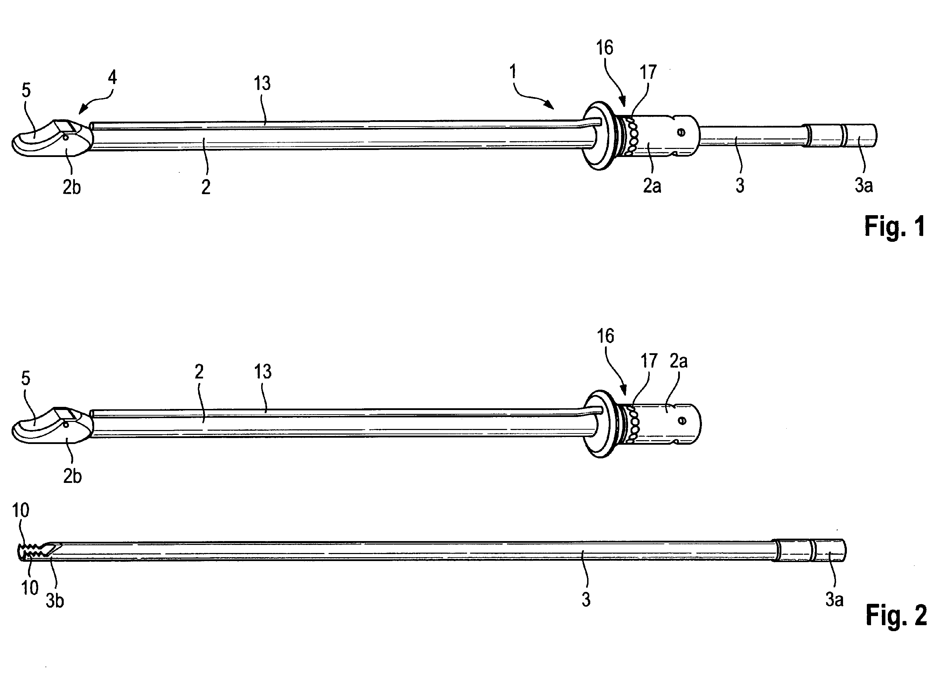

Nachfolgend wird unter Bezugnahme auf die Fig. 1 bis 10 ein chirurgisches Instrument 1 gemäß einem bevorzugten Ausführungsbeispiel der Erfindung im Detail beschrieben.

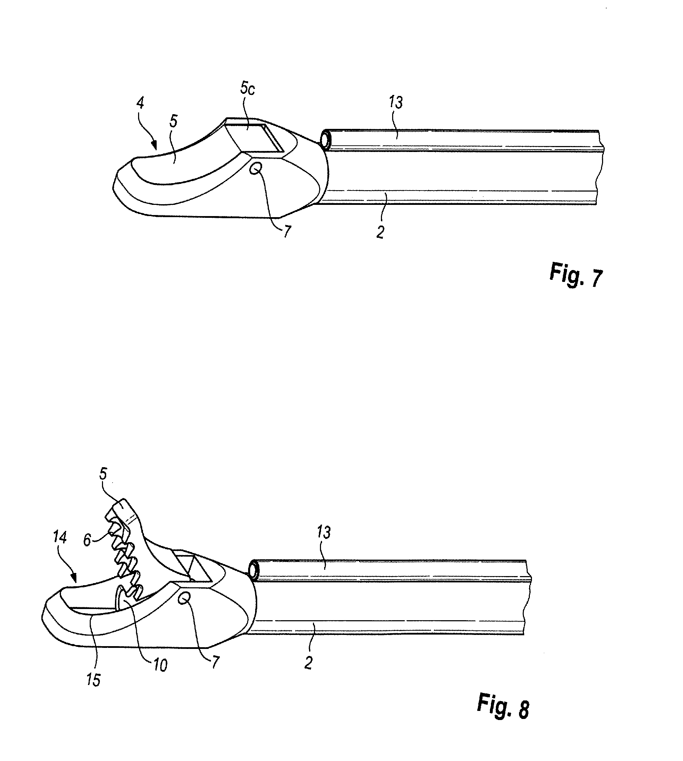

Wie aus den Fig. 1 und 2 ersichtlich ist, umfasst das chirurgische Instrument 1 ein Außenrohr 2 und ein im Außenrohr angeordnetes Innenrohr 3. Wie aus Fig. 2 ersichtlich ist, kann das Innenrohr 3 vollständig aus dem Außenrohr entnommen werden. An einem distalen Ende 2b des Außenrohrs 2 ist eine Schneidvorrichtung 4 in Form einer Stanze angeordnet. Die Schneidvorrichtung 4 umfasst einen schwenkbaren Schneidbacken 5 und einen mit den schwenkbaren Schneidbacken 5 zusammenwirkenden statischen Schneidbacken 15 (vergleiche Fig. 8 bis 10). Am entgegengesetzten proximalen Ende 2a des Außenrohrs 2 ist eine Kupplung 16, umfassend eine Vielzahl von Bohrungen 17, angeordnet. Die Kupplung 16 dient zur lösbaren Verbindung mit einem nicht dargestellten Handstück. Das Handstück steht mit einem proximalen Ende 3a des Innenrohrs 3 in Wirkverbindung, um eine axiale Bewegung des Innenrohrs 3 innerhalb des Außenrohrs 2 nach einer Betätigung am Handstück auszuführen.

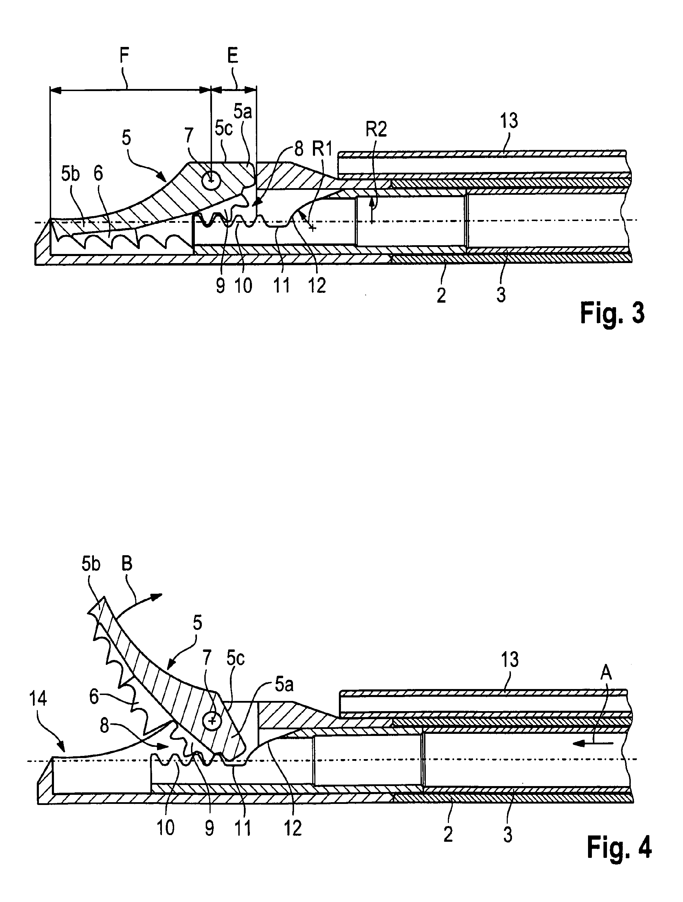

Die Fig. 3 bis 6 zeigen im Detail das distale Ende des chirurgischen Instruments 1. Wie aus den Fig. 3 bis 6 ersichtlich ist, ist die Schneidbacke 5 der Schneidvorrichtung 4 um eine Drehachse 7 schwenkbar angeordnet. Zwischen dem Innenrohr 3 und der Schneidbacke 5 am Außenrohr 2 ist eine lösbare Verzahnungsverbindung mit einem Zahnradelement 9 und einer Zahnstange 10 vorgesehen. Das Zahnradelement 9 ist an der schwenkbaren Schneidbacke 5 angeordnet und die Zahnstange 10 ist am distalen Ende 3b des Innenrohrs 3 gebildet (vergleiche auch Fig. 2). Im eingeschobenen Zustand des Innenrohrs 3 in das Außenrohr 2 kommt die Zahnstange 10 mit dem Zahnradelement 9 an der Schneidbacke 5 in Eingriff, so dass durch eine Axialbewegung des Innenrohrs 3 über die Verzahnungsverbindung 8 die Schneidbacke 5 um die Drehachse 7 geschwenkt werden kann. Dadurch kann die Schneidvorrichtung 4 geöffnet und geschlossen werden, wenn das Innenrohr 3 in Axialrichtung hin- und herbewegt wird. An der Schneidbacke 5 sind ferner mehrere Schneiden 6 angeordnet, so dass durch ein Schwenken der Schneidbacke 5 Körpergewebe o.Ä. abgetrennt und gegebenenfalls gegriffen werden kann. Dabei bildet die Schneidvorrichtung 4 im geöffneten Zustand (Fig. 4) eine Öffnung 14, in welche das abgetrennte Gewebe aufgenommen werden kann. Dieses Gewebe kann dann durch das hohle Innenrohr 3 mittels einer am distalen Ende 3a anliegenden Saugströmung von der Öffnung 14 weggesaugt werden.

Wie weiter insbesondere aus den Fig. 3 bis 6 ersichtlich ist, weist das Innenrohr 3 unmittelbar benachbart zur Zahnstange 10 eine Ausnehmung 11 und eine Auflaufschräge 12 auf. Weiterhin ist an der Außenseite des Außenrohrs 2 ein Spülrohr 13 angeordnet, mit welchem bei Bedarf eine Spülflüssigkeit zugeführt werden kann.

Wie aus den Fig. 3 bis 6 ersichtlich ist, ist die Drehachse 7 an der schwenkbaren Schneidbacke 5 derart angeordnet, dass sie diese in einen distalen Bereich 5b und einen proximalen Bereich 5a unterteilt. Dabei ist der distale Bereich 5b (Länge F) länger als der proximale Bereich 5a (Länge E) ausgebildet. Am proximalen Bereich 5a ist ferner eine Kontaktfläche 5c angeordnet, welche mit der Auflaufschräge 12 am Innenrohr 3 in Kontakt bringbar ist. Die Auflaufschräge 12 weist dabei einen durch einen Radius R1 gebildeten Bereich auf, der in einen geraden Bereich übergeht. Ein Verhältnis des Radius R1 zu einem Radius R2 eines Außendurchmessers des Innenrohrs 3 beträgt dabei 1,49.

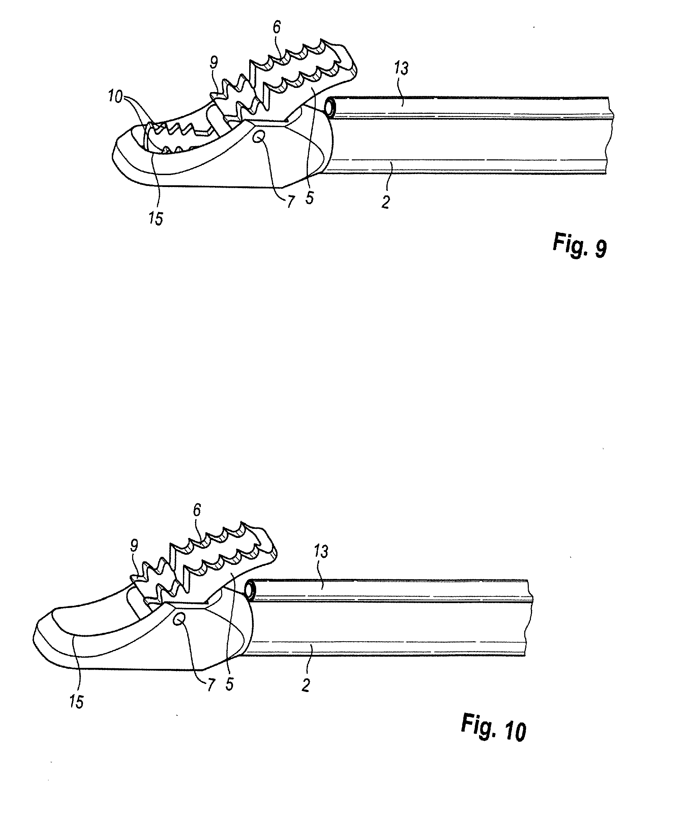

Die Funktion des erfindungsgemäßen chirurgischen Instruments 1 ist dabei wie folgt. Im eingebauten Zustand ist das Innenrohr 3 in das Außenrohr 2 eingeschoben und die Verzahnungsverbindung 8 zwischen dem Zahnradelement 9 und der Zahnstange 10 befindet sich im Eingriff. Wenn nun das Innenrohr 3 beispielsweise mittels eines manuell betätigbaren Handstücks in Axialrichtung hin- und herbewegt wird, wird auch die Zahnstange 10 linear bewegt und dadurch öffnet und schließt die schwenkbare Schneidbacke 5 durch einen Schwenkvorgang um die Drehachse 7. In Fig. 4 ist mit dem Pfeil A angedeutet, dass das Innenrohr in Axialrichtung zum distalen Ende hin bewegt wird, wodurch die schwenkbare Schneidbacke 5 um die Drehachse 7 in Richtung des Pfeils B geschwenkt wird und die Schneidvorrichtung 4 somit geöffnet wird. Durch diese Öffnungs- und Schließbewegung der Schneidbacke 5 kann beispielsweise Gewebe während einer Operation entfernt werden. Das Gewebe wird durch die Schneidbacke 5 abgeschnitten und über die Öffnung 14 und das hohle Innenrohr 3 abgesaugt. Wie insbesondere aus Fig. 2 und 9 ersichtlich, ist die Zahnstange 10 am distalen Ende des Innenrohrs 3 nur an den Randbereichen gebildet, so dass sich zwischen den beiden Zahnreihen, welche die Zahnstange 10 bilden, ein Freiraum befindet, über welchen das abgetrennte Gewebe und Blut oder andere Flüssigkeiten abgesaugt werden können.

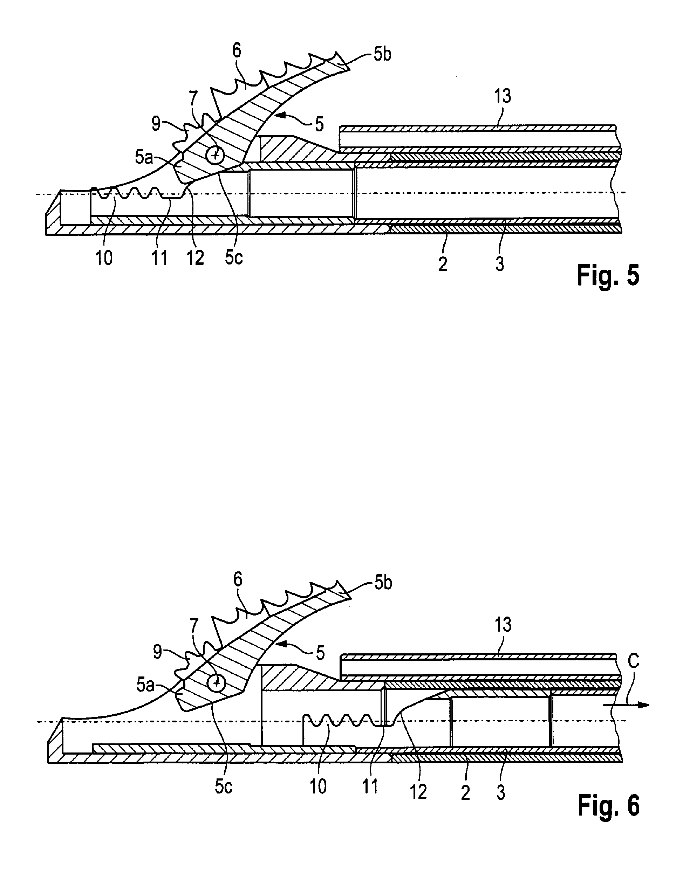

Aufgrund der Bewegbarkeit des Innenrohrs 3 im Außenrohr 2 ist ein kleiner Spalt zwischen einer Außenseite des Innenrohrs 3 und einer Innenseite des Außenrohrs 2 gebildet. In diesem Spalt könne sich Blut, Flüssigkeiten oder kleine Gewebereste ansammeln. Um nun eine verbesserte Reinigungsmöglichkeit bereitzustellen, kann das Innenrohr 3 vom Außenrohr 2 entnommen werden. Hierzu wird die Verzahnungsverbindung 8 außer Eingriff gebracht. Dies erfolgt dadurch, dass ausgehend von dem in Fig. 4 gezeigten Zustand das Innenrohr 3 weiter in Richtung des Pfeils A bewegt wird, so dass die Schneidbacke 5 sich noch weiter öffnet. Dadurch wird das Ende des proximalen Bereichs 5a in die Ausnehmung 11 am Innenrohr 3 aufgenommen und die Auflaufschräge 12 kommt mit der Kontaktfläche 5c der Schneidbacke 5 in Kontakt. Durch diesen Kontakt zwischen der Auflaufschräge 12 und der Kontaktfläche 5c wird die Schneidbacke 5 noch weiter in Öffnungsrichtung geschwenkt, was in Fig. 5 dargestellt ist. Das Schwenken der Schneidbacke 5 wird dabei so lange ausgeführt, bis das Innenrohr 3 ohne mit der geöffneten Schneidbacke 5 zu kollidieren aus dem Außenrohr 2 herausgezogen werden kann. Dieser Zustand ist in Fig. 5 erreicht und das Heranziehen ist in Fig. 6 durch den Pfeil C angedeutet.

Dadurch wird das Innenrohr 3 vom Außenrohr 2 gelöst, so dass beide Bauteile separat gereinigt werden können. Nach einer erfolgten Reinigung kann das Innenrohr 3 wieder in das Außenrohr 2 eingeführt werden, wobei sich die Schneidbacke 5 vorzugsweise in der in Fig. 6 gezeigten Position befindet. Dabei wird das Innenrohr 3 vollständig in das Außenrohr 2 eingeschoben und dann wird manuell die geöffnete Schneidbacke 5 geschlossen, wodurch das Innenrohr 3 über die Kontaktfläche 5c und das Ende des proximalen Bereichs 5a, welches während des Schwingvorgangs in der Ausnehmung 11 aufgenommen wird, zurückgeschoben, bis das Zahnradelement 9 mit der Zahnstange 10 in Eingriff kommt. Dadurch ist das chirurgische Instrument wieder einsatzfähig.

Erfindungsgemäß kann somit ein chirurgisches Instrument 1 bereitgestellt werden, welches eine Schneidvorrichtung 4 an einem distalen Ende aufweist und über ein hohles Innenrohr 3 eine Absaugung von Blut, Flüssigkeiten, Gewebe usw. ermöglicht. Dabei kann das Innenrohr nach einem Einsatz vom Außenrohr gelöst werden und eine separate Reinigung der beiden Bauteile erfolgen. Anschließend wird das Innenrohr 3 wieder in das Außenrohr 2 eingeschoben und kann für eine nächste Operation verwendet werden. Die lösbare Verbindung zwischen dem Innenrohr 3 und dem Außenrohr 2, wird dabei über die lösbare Verzahnungsverbindung 8 hergestellt. Der Aufbau des chirurgischen Instruments bleibt dabei trotzdem sehr einfach und kostengünstig und stellt ferner eine ausfallfreie Betätigung der Schneidvorrichtung 4 sicher.

### Bezugszeichenliste

- 1: Chirurgisches Instrument
- 2: Außenrohr
- 2a: Proximales Ende
- 2b: Distales Ende
- 3: Innenrohr
- 3a: Proximales Ende
- 3b: Distales Ende
- 4: Schneidvorrichtung
- 5: Schwenkbare Schneidbacke
- 5a: Proximaler Bereich
- 5b: Distaler Bereich
- 5c: Kontaktfläche
- 6: Schneide
- 7: Drehachse
- 8: Verzahnungsverbindung
- 9: Zahnradelement
- 10: Zahnstange
- 11: Ausnehmung
- 12: Auflaufschräge
- 13: Spülrohr
- 14: Öffnung
- 15: Stationäre Schneidbacke
- 16: Kupplung
- 17: Bohrung
- R1: Radius Auflaufschräge
- R2: Radius Außendurchmesser Innenrohr

## Patentansprüche

1. Chirurgisches Instrument, umfassend
- ein Außenrohr (2),
- ein Innenrohr (3), welches beweglich im Außenrohr (2) angeordnet ist und vollständig vom Außenrohr (2) lösbar ist,
- eine chirurgische Schneidvorrichtung (4) zum Abtrennen und/oder Greifen von Körpergewebe oder dergleichen, und
- eine lösbare Verzahnungsverbindung (8) mit einem Zahnradelement (9) und einer Zahnstange (10)
- wobei die Schneidvorrichtung (4) eine schwenkbare Schneidbacke (5) und eine stationäre Schneidbacke (15) umfasst, und die schwenkbare Schneidbacke (5) um eine Achse (7) schwenkbar am Außenrohr (2) angeordnet ist,
- wobei die schwenkbare Schneidbacke (5) mittels der Verzahnungsverbindung (8) mit dem Innenrohr (3) verbunden ist und durch eine axiale Bewegung des Innenrohrs (3) betätigbar ist, und
- wobei das abgetrennte Gewebe durch das Innenrohr (3) hindurch absaugbar ist.

2. Chirurgisches Instrument nach Anspruch 1, **dadurch gekennzeichnet, dass** die Zahnstange (10) am Innenrohr (3) und das Zahnradelement (9) an der schwenkbaren Schneidbacke (5) angeordnet ist.

3. Chirurgisches Instrument nach einem der vorhergehenden Ansprüche, **dadurch gekennzeichnet, dass** die schwenkbare Schneidbacke (5) einen distalen Bereich (5b) und einen proximalen Bereich (5a) aufweist, wobei am distalen Bereich (5b) mindestens eine Schneide (6) angeordnet ist und der proximale Bereich (5a) eine Länge (E) aufweist, welche kürzer als eine Länge (F) des distalen Bereichs (5b) ist.

4. Chirurgisches Instrument nach einem der vorherigen Ansprüche, **dadurch gekennzeichnet, dass** am Innenrohr (3) ein Kontaktbereich (12) und am proximalen Bereich (5a) der Schneidbacke eine Kontaktfläche (5c) ausgebildet ist, welche miteinander in Kontakt bringbar sind.

5. Chirurgisches Instrument nach Anspruch 4, **dadurch gekennzeichnet, dass** der Kontaktbereich (12) am Innenrohr (3) als Auflaufschräge, insbesondere als ballige Auflaufschräge, ausgebildet ist.

6. Chirurgisches Instrument nach Anspruch 4 oder 5, **dadurch gekennzeichnet, dass** zwischen der Zahnstange (10) und dem Kontaktbereich (12) eine Ausnehmung (11) zur Aufnahme des proximalen Bereichs (5a) der Schneidbacke (5) bei einem Schwenkvorgang angeordnet ist.

7. Chirurgisches Instrument nach einem der vorhergehenden Ansprüche, ferner umfassend ein Spülrohr (13), welches an einer Außenseite des Außenrohrs (2) angeordnet ist.

8. Chirurgisches Instrument nach einem der vorhergehenden Ansprüche, ferner umfassend eine Kupplung (16), welche am Außenrohr (2) angeordnet ist, um eine Verbindung mit einem Handstück herzustellen.

9. Chirurgisches Instrument nach Anspruch 8, **dadurch gekennzeichnet, dass** die Kupplung (16) eine Vielzahl von Bohrungen (17) entlang ihres Außenumfangs aufweist.

10. Chirurgische Anordnung umfassend ein chirurgisches Instrument nach einem der vorhergehenden Ansprüche und ein mit dem chirurgischen Instrument verbundenes Handstück.
